(19) Europäisches Patentamt
European Patent Office
Office européen des brevets

(11) EP 0 539 256 B1

(12) FASCICULE DE BREVET EUROPEEN

(45) Date de publication et mention de la délivrance du brevet:
**08.01.1997 Bulletin 1997/02**

(51) Int Cl.[6]: **C08G 75/00**, C07C 329/16

(21) Numéro de dépôt: **92402721.2**

(22) Date de dépôt: **06.10.1992**

# (54) Polymères à groupements disulfure de xanthate et leur procédé de préparation

Polymere mit Xanthatdisulfid-Gruppen und Verfahren zu ihrer Herstellung

Polymers with xanthate disulphide groups and process for their preparation

(84) Etats contractants désignés:
**AT DE ES FR GB IE IT NL SE**

(30) Priorité: **23.10.1991 FR 9113082**

(43) Date de publication de la demande:
**28.04.1993 Bulletin 1993/17**

(73) Titulaire: **RHONE-POULENC CHIMIE**
**92408 Courbevoie Cédex (FR)**

(72) Inventeurs:
- **Charmot, Dominique**
**F-93310 Le Pré Saint Gervais (FR)**
- **Clouet, Gilbert**
**F-67610 La Wantzenau (FR)**
- **Corpart, Pascale**
**F-60180 Nogent sur Oise (FR)**

(74) Mandataire: **Seugnet, Jean Louis et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 342 073**
**EP-A- 0 450 492**
**EP-A- 0 418 118**
**US-A- 5 011 965**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 539 256 B1

## Description

La présente invention concerne des composés dits macroxanthates et polyxanthates, leurs procédés de préparation et leurs utilisations en polymérisation radicalaire en milieu monophasique de monomères vinyliques.

Les copolymères blocs contenant des séquences hydrophobes en alternance avec des séquences hydrophiles sont aujourd'hui tout particulièrement recherchés pour stabiliser des dispersions aqueuses.

A titre d'exemple de composés types de cette nouvelle famille de copolymères on peut notamment citer les copolymères blocs à base de styrène et d'oxyde d'éthylène.

La préparation de ces copolymères est habituellement réalisée par polymérisation anionique ou polymérisation radicalaire faisant appel à la technique des iniferters (J. Macromol. Sci. Rev. Macromol. Chem. Phys., 1991, C31(2-3), 311).

Cette technique iniferter met en application, dans la polymérisation radicalaire de monomères vinyliques comme le styrène, la triple fonction d'initiateur, d'agent de transfert de chaîne et d'agent de terminaison, des groupes disulfures de thiurame, incorporés dans une chaîne macromoléculaire comme le polyoxyde d'éthylène par exemple. Cette triple fonction des disulfures de thiurame a été décrite dans la revue Makromol.Chem.Rapid.Commun.3, 127-132 (1982).

C'est ainsi que dans le cas particulier du polyoxyde d'éthylène, les dérivés disulfures de macro- ou poly-thiurame correspondants, encore désignés sous les noms macro- ou poly-iniferter, sont obtenus à partir de l'oligomère polyoxyde d'éthylène terminé à une ou deux extrémités de sa chaîne par une fonction alcool.

Leurs préparations impliquent la transformation chimique de la ou les fonctions hydroxyles en un ou des groupement(s) amine(s) secondaire(s).
L'extension de chaîne de cette mono- ou di-amine par une réaction de dithiocarbamylation suivie d'un couplage par voie oxydante, conduit au macro- ou poly-iniferter attendu.

Les brevets EP-A-O 342 073 et EP-A-O 418 118 décrivent en particulier la préparation de polymères de ce type incorporant des groupements disulfures de thiurame.

La polymérisation thermique radicalaire de composés vinyliques, en présence de ces macro- ou poly-iniferters, conduit ultérieurement à la formation des copolymères séquencés amphiphiles recherchés.

La présente invention a précisément pour objet de nouveaux composés susceptibles de remplacer avantageusement dans la polymérisation radicalaire de composés vinyliques les composés macro- et poly-iniferter précités.

Selon la présente invention, il a été cherché à préparer de nouveaux composés exempts de groupements disulfures de thiurame mais qui soient néanmoins dotés de propriétés de transfert de chaînes.

Ce but est atteint par la présente invention grâce à l'utilisation de disulfures de xanthate.

Les disulfures de xanthate sont des produits connus. Ils ont essentiellement été utilisés pour leur fonction d'agent de transfert dans des polymérisations telles que celles du butadiène ou du chloroprène. Toutefois, ces disulfures de xanthate ont toujours été obtenus à partir d'alcools courts de $C_1$ à $C_8$.

De manière inattendue, ces disulfures de xanthate sont formés efficacement selon l'invention à partir des fonctions alcools portées par des chaînes de polymères.

Les nouveaux agents de transfert à base polymère selon l'invention, encore appelés ci-après macro- ou polyxanthates, sont obtenus en faisant réagir directement le disulfure de carbone sur le ou les alcoolates de la chaîne macromoléculaire considérée.

Ceci conduit donc à la formation de liaisons O-C au sein de la chaîne macromoléculaire ainsi transformée.

La substitution de liaisons O-C aux liaisons N-C, habituellement obtenues avec les disulfures de thiurame, est particulièrement avantageuse.

Tout d'abord, il s'agit d'une simplification de procédé. Dans le cas des disulfures de thiurame, il était au préalable nécessaire de transformer les fonctions hydroxyles de la chaîne macromoléculaire en leurs fonctions amines correspondantes.

Par ailleurs, en ce qui concerne plus particulièrement le polyxanthate ainsi obtenu, il possède une tenue thermique supérieure aux composés iniferter du fait de la stabilité supérieure conférée au lien disulfure par la présence de l'atome d'oxygène.

Enfin, du fait de leur stabilité accrue, ces macro- ou poly-xanthates ne peuvent jouer le rôle d'amorceur lors de leur emploi ultérieur dans la polymérisation de monomères vinyliques. Il est donc nécessaire d'amorcer en leur présence, la réaction de polymérisation avec un initiateur externe.

La présence de cet initiateur a pour avantage de rendre plus aisé le contrôle de la réaction de polymérisation et en particulier d'en accroître considérablement la vitesse par rapport à celle ordinairement atteinte avec les iniferters.

La présente invention a donc pour premier objet des poly- et macro-xanthates c'est à dire des chaînes macromoléculaires incorporant au moins un groupement disulfure de xanthate.

La présente invention a également pour objet des procédés de préparation de ces poly- et macro-xanthates à base polymère.

Enfin la présente invention se rapporte en outre à l'utilisation de ces poly- et macro-xanthates à base polymère

dans la polymérisation radicalaire en milieu monophasique de composés vinyliques.

Plus précisément la présente invention se rapporte à de nouveaux composés chimiques représentés par les formules générales

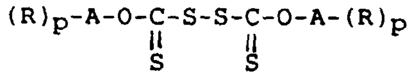 (I)

ou

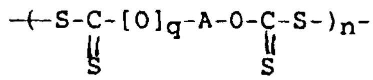 (II)

dans lesquelles
A représente:

- une séquence polymère issue d'au moins un monomère choisi parmi les monomères vinyliques et diéniques comme le styrène et le butadiène par exemple, auquel cas p vaut zéro et q est égal à 1 ou bien
- une séquence polyoxyalkylène, chaque chaînon alkylène ayant au plus 6 atomes de carbone, auquel cas p est égal à 1 et q vaut zéro, avec n variant entre 2 à 30 et de préférence entre 3 et 15 et R représentant un reste alkyle, cycloalkyle ou aryle.

Par macroxanthate, on désigne selon l'invention les polymères xanthates contenant un seul groupement disulfure de xanthate dans leur chaîne principale c'est à dire les composés de formule générale I.

Quant au terme polyxanthate, il est utilisé pour désigner les polymères xanthates représentés par la formule générale II c'est à dire comportant plus d'un groupement disulfure de xanthate dans leur chaîne principale.

Parmi les composés, objets de l'invention, on citera tout particulièrement les composés de formule générale I ou II dans lesquelles A représente une séquence polyoxyalkylène.

Cette séquence polyoxyalkylène est de préférence choisie parmi les séquences des formules suivantes :

$$-(-O-CH_2-CH_2-)_r-$$

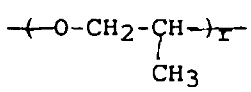

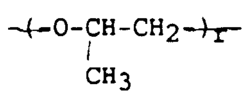

et

$$-(-O-(CH_2)_4-)_r-$$

où r vaut de 1 à 1000 et de préférence de 1 à 200.

Pour des raisons de commodité A pourra être désigné ci-après sous l'appellation de polymère.

La présente invention se rapporte tout particulièrement aux composés de formules générales I ou II dans lesquelles la séquence polyoxyalkylène, représentée par A, dérive d'un polyoxyde d'éthylène dont la masse moléculaire varie de

préférence entre 1000 et 10 000.

Il s'agit de préférence du polyoxyde d'éthylène de masse moléculaire supérieure à environ 3000 qui est le premier de cette série de masse à se présenter sous forme pulvérulente. Il s'en suit une récupération plus aisée du produit de la réaction.

La présente invention se rapporte également à un procédé de préparation des composés de formules générales I ou II.

Classiquement, les disulfures de xanthates sont obtenus par réaction d'un alcoolate sur le disulfure de carbone. Cette réaction conduit à la formation d'un sel de xanthate qui peut être oxydé pour aboutir au disulfure de xanthate attendu.

Les composés, objets de l'invention, peuvent être préparés par réaction d'un alcoolate de formules générales

$$(R)_p\text{-A-}O^{\ominus}\ (X) \qquad \text{(Ibis)}$$

ou

$$(X)^{\ominus}(O)_q\text{-A-}O^{\ominus}(X) \qquad \text{(IIbis)}$$

dans lesquelles A, R, p et q ont les significations ou valeurs explicitées çi-dessus et X représente un sel métallique et de préférence un atome de potassium ou de sodium, sur le disulfure de carbone, en présence d'un agent oxydant ou ajoutant cet agent à la fin de la réaction.

L'obtention des macro- et poly-xanthates débute donc par la transformation en alcoolate(s) de la ou des fonctions alcools d'un polymère, terminé à une ou aux deux extrémités de sa chaîne par une fonction alcool.

Deux modes de préparation des alcoolates ont été mis en oeuvre dans le cadre de la présente invention.

Selon un premier procédé, les alcoolates peuvent être obtenus par une réaction d'oxydo-réduction. A cet effet, on utilise de préférence, à titre ae réducteur, l'hydrure de sodium. L'addition de l'hydrure de sodium au dérivé alcool se fait graduellement et l'alcoolate attendu est récupéré en fin de réaction.

Cette transformation des alcools peut également se faire en milieu aqueux par action d'une base en présence d'un fort excès de groupements alcool.

Toutefois, dans le cadre de la présente invention, il a été nécessaire d'apporter des modifications importantes à ce second procédé.

Selon l'invention, l'alcoolate de polymère est préparé par réaction d'un polymère de formules générales :

$$(R)_p\text{-A- }OH \qquad \text{(Iter)}$$

ou

$$H\text{-}(O)_q\text{-A-}OH \qquad \text{(IIter)}$$

avec A, R, p et q tels que définis auparavant, en milieu hétérogène avec un fort excès d'une base forte qui est de préférence la soude ou la potasse.

En fait, deux modes opératoires différents sont préconisés selon l'invention dans le cas de ce second procédé.

Le premier mode consiste à dissoudre le polymère de formule générale Iter ou IIter dans un solvant organique polaire ou non et à y ajouter la base sous forme d'une solution aqueuse saturée.

Les solvants convenables peuvent être notamment le THF, le toluène ou le DMF.

L'alcoolate, ainsi formé, subit ensuite la réaction de dithiocarbamylation par ajout de $CS_2$.

Généralement, les concentrations en alcoolates et $CS_2$ sont respectivement pour l'alcoolate comprises entre $10^{-3}$ à $5.10^{-1}$ moles par litre et pour $CS_2$ entre $10^{-1}$ et $5.10^{-1}$ moles par litre. Ce qui correspond environ à un rapport $CS_2$-fonction alcoolate de l'ordre de 3.

Classiquement, l'oxydation est réalisée ultérieurement par ajouts graduels d'une solution oxydante.

L'agent oxydant utilisé dans cette seconde étape est choisi par exemple parmi l'iode, l'eau oxygénée, les hypochlorites d'alkyle comme l'hypochlorite de tert-butyle et les hydroperoxides d'alkyle et d'aryle. Cet oxydant est utilisé en léger excès par rapport à la concentration en alcoolate.

Il s'agit de préférence d'une solution d'iode qui permet de contrôler précisément le déroulement de l'oxydation.

Dans ce cas, l'oxydation est achevée à la persistance de la coloration de l'iode.

La seconde méthode de préparation de l'alcoolate est qualifiée, selon l'invention, de synthèse en masse car elle est réalisée en absence de tout solvant.

Le polymère de formule générale Iter ou IIter est porté à au moins sa température de fusion en présence uniquement de la base à l'état solide.

Dans le cas particulier de la potasse ou de la soude , elles peuvent être directement introduites dans le polymère en fusion sous la forme de leurs pastilles. Une autre variante consiste à lyophiliser au préalable une solution aqueuse de ces bases dans le récipient où doit être ensuite fondu le polymère. Il est également possible de les utiliser sous forme d'une poudre finement broyée.

Dans le cas de cette seconde méthode, l'ensemble du milieu réactionnel est maintenu à au moins la température de fusion du polymère considéré pendant au moins dix heures. A la fin de la réaction le mélange est refroidi, le résidu de base éventuellement éliminé et le milieu récupéré avec un solvant organique.

Les réactions de dithiocarbamylation et d'oxydation sont ensuite effectuées conformément à ce qui a été décrit pour le premier mode de la seconde méthode décrite auparavant pour la préparation des alcoolates.

Lors de l'oxydation ultérieure on note en particulier, dans le cas où la réaction d'alcoylation à été réalisée en présence d'un fort excès de base, l'apparition d'une phase visqueuse.

A l'issue de cette oxydation, les macro- ou poly-xanthates obtenus peuvent être récupérés selon différentes manières, choisies en fonction du déroulement de l'oxydation.

Lorsque le milieu réactionnel demeure monophasique, le xanthate à base polymère de formule générale I ou II obtenu, en est extrait par précipitation dans un solvant convenable.

En revanche, si la phase visqueuse précédemment citée apparaît, il convient, dans l'éventualité où le solvant serait miscible avec l'eau, d'évaporation celui-ci et de reprendre le résidu dans de l'eau.

Le macro- ou poly-xanthate isolé peut être ensuite purifié, par exemple par dialyse ou ultrafiltration dans le cas de grandes quantités. L'ultrafiltration présente en particulier l'avantage de conduire à un produit débarrassé des principales impuretés du type sels minéraux. Le polymère est ensuite récupéré par lyophilisation de la phase aqueuse propre.

L'objet de la présente invention s'étend à tout produit susceptible d'être obtenu par la mise en oeuvre d'un procédé tel que décrit précédemment.

La présente invention a également pour objet l'utilisation des composés de formule I ou II comme agent "ferter" c'est à dire un composé ayant la fonction d'agent de transfert de chaîne et d'agent de terminaison, dans la polymérisation radicalaire de monomères vinyliques en milieu monophasique.

Par polymérisation en milieu monophasique on entend désigner selon l'invention les polymérisations où le monomère et le composé de formule générale I ou II sont mélangés au sein d'un même solvant et également les polymérisations où le composé de formule générale I ou II est directement introduit dans le monomère sans apport d'un solvant quelconque.

Les composés de formules I ou II sont appelés "ferter" car, comme on l'a explicité précédemment, ils ne possèdent pas la fonction d'initiateur de polymérisation à la différence des poly-disulfures de thiurame.

Comme monomères vinyliques utilisables on peut notamment citer :

- les monomères acryliques et méthacryliques choisis plus particulièrement parmi l'acide acrylique ou méthacrylique, les acrylates et les méthacrylates d'alkyle en $C_1$-$C_{12}$ tels que le méthacrylate de méthyle, le méthacrylate de butyle,
- les monomères styrènes tels que le styrène, l'alpha-méthylstyrène, le tert-butyl-styrène et le vinyltoluène,
- les acrylamides.
- les acides fumarique ou maléïque,
- les diènes tels que le butadiène et le chloroprène.

Les monomères vinyliques préférés sont le styrène et les (méth)acrylates d'alkyle.

Dans le cas présent, il est nécessaire d'introduire un initiateur de polymérisation du type peroxyde d'alkyle, peroxydicarbonates ou AIBN (azobisisobutyronitrile). En ce qui concerne plus particulièrement l' AIBN, il est de préférence utilisé en une quantité comprise entre $10^{-4}$ et $10^{-2}$ moles par litre.

Le monomère, additionné des quantités convenables en poly- ou macro-xanthate et initiateur de polymérisation, est placé sous vide à une température appropriée à la polymérisation.

La quantité de macro- ou poly-xanthate introduite pour une polymérisation est pour des raisons de commodité définie par la concentration en liens disulfure de xanthate introduite. Cette concentration en liens disulfure de xanthate varie généralement entre $10^{-4}$ à $10^{-1}$ moles par litre.

La polymérisation radicalaire conduit à l'insertion du bloc polyvinylique entre les deux atomes de soufre du polyxanthate. Le poids moléculaire des blocs polyvinyliques dépend du rapport molaire monomère vinylique/macro- ou

poly-xanthate.

Le polymère formé peut être isolé par précipitation dans un non-solvant qui solubilise en revanche le polyxanthate initial. Il est ensuite récupéré par filtration et séché.

La polymérisation de monomères vinyliques en présence de polyxanthates conduit à l'obtention de polymères multiblocs dotés de propriétés amphiphiles.

On note que lorsque la concentration en polyxanthate augmente la vitesse de polymérisation décroît. De même les masses moléculaires décroissent avec des teneurs croissantes en polyxanthate.

La présente invention se rapporte également aux polymères vinyliques susceptibles d'être obtenus par polymérisation radicalaire d'au moins un monomère vinylique en milieu monophasique et en présence d'au moins un composé de formule générale I ou II.

Pour mieux illustrer l'objet de l'invention on en décrit ci-après plusieurs exemples de modes de réalisation qui sont donnés à titre indicatif et non limitatif.

Les masses moléculaires des polymères sont mesurées par G.P.C.. Sauf indications contraires les pourcentages et parties dans ce qui suit ou ce qui précède sont exprimés en poids.

Dans ces exemples, on a utilisé les abréviations suivantes :

- t : temps de réaction
- X : taux de conversion

$$X = \frac{(m_{MMA})_o - (m_{MMA})}{(m_{MMA})_o}$$

- Mω : masse moléculaire moyenne en poids
- Mn : masse moléculaire moyenne en nombre
- Rpo : vitesse de polymérisation initiale
- Xpx : taux du polyxanthate initial incorporé dans le copolymère :

$$Xpx = \frac{(m_{px})o - (m_{px})}{(m_{px})o}$$

**A - PREPARATION D'UN POLYXANTHATE EN SOLUTION**

**EXEMPLE 1** :

2 g de POE ($M_{POE}$ = 3400, difonctionnel) soit 1,18.$10^{-3}$ moles de fonctions OH, sont dissous dans 10 ml de THF. A cette solution sont additionnés 0,07 ml (1,25 $10^{-3}$ moles de KOH) d'une solution aqueuse de potasse à 1 g/ml. Après quatre à cinq heures, on ajoute 0,2 ml (3,32 $10^{-3}$ moles) de $CS_2$. Le milieu prend immédiatement une coloration jaune.

L'oxydation est commencée une demi-heure environ après l'adjonction de $CS_2$. Elle se fait par ajouts graduels d'une solution d'iode dans le THF, jusqu'à persistance de la coloration de l'iode.

Le polyxanthate est récupéré par précipitation directe dans l'éther et analysé par G.P.C. Il apparaît sur le chromatogramme une trainée dans les grandes masses significative d'un léger couplage qui aboutit à une : Mω (polyxanthate) égale à 4100 contre une Mω (POE initial) de 3400.

**EXEMPLE 2** :

L'exemple 2 est réalisé conformément au mode opératoire décrit en exemple 1 mais avec une quantité de potasse aqueuse supérieure. Dans ce cas on emploie 0,3 ml soit 5,36 $10^{-3}$ moles de KOH.

Cet excès de KOH conduit à la démixion d'une phase visqueuse lors de l'oxydation à l'iode.

La récupération du polyxanthate se fait donc, après évaporation du THF, par reprise dans l'eau, dialyse à travers une membrane poreuse, de masse de coupure 500 et lyophilisation. L'analyse G.P.C aboutit à une Mω (polyxanthate) égale à 13 900 contre une Mω (POE initial) de 3400.

Le couplage est beaucoup plus important que dans l'exemple 1.

## B - PREPARATION D'UN POLYXANTHATE EN MASSE

**EXEMPLE 3** :

- POE ($M_{POE}$ = 3400 difonctionnel) = 2 g soit 1,18 x $10^{-3}$ moles de fonctions OH
- KOH = 0,1 g soit 1,78 $10^{-3}$moles
- $CS_2$ = 0,2 ml soit 3,32 x $10^{-3}$ moles
- THF = 10 ml

Dans ce cas, on commence par fondre à 70°C, le POE. Puis on ajoute la potasse directement sous forme de pastilles. L'ensemble est maintenu à température pendant au moins une dizaine d'heures. La potasse résiduelle est alors retirée et le milieu est refroidi puis repris dans le THF. Le $CS_2$ est ensuite additionné. La solution vire au jaune. Un demi-heure environ après l'addition du $CS_2$, on réalise l'oxydation à l'iode comme décrit dans l'exemple 1.

Cette dernière réaction est poursuivie jusqu'à persistance de la coloration violette de $I_2$.

Le polymère est récupérée par précipitation dans l'éther et analysé par G.P.C.. Le chromatogramme obtenu montre une double distribution représentative d'un mélange, du produit juste couplé (deux séquences POE) et du produit initial.

La masse moyenne Mω(polyxanthate) est égale à 4600 contre une Mω (POE initial) de l'ordre de 3400.

## C- PREPARATION D'UN POLY(MACRO)XANTHATE EN MASSE

**EXEMPLE 4:**

Cet essai est semblable à celui de l'exemple 3 si ce n'est que la quantité de potasse utilisée est plus importante. On en utilise 0,3 g soit 5,36 x $10^{-3}$ moles. La réaction se déroule exactement comme pour l'exemple 3 mais conduit à un produit bien mieux couplé puisque l'on obtient une masse moyenne Mω (polyxanthate) de l'ordre 9400 contre une Mω (POE initial) de l'ordre de 3400.

**EXEMPLE 5:**

Cet essai est réalisé selon le mode opératoire et avec les quantités décrites en exemple 4 mais la potasse n'y est pas employée sous forme de pastilles. Elle est préalablement dispersée dans le ballon de réaction par lyophilisation d'une solution aqueuse de KOH.

L'excès de base reste donc dans le milieu réactionnel jusqu'à la fin de la préparation. L'oxydation aboutit dans ce cas à la démixion d'une phase visqueuse déjà présente dans l'exemple 2. De la même facon qu'en l'exemple 2, on récupère alors le polyxanthate, par dialyse puis lyophilisation.

La meilleure dispersion de la potasse ou son excès rémanant favorisent la réaction puisque l'on obtient une masse moyenne Mω (polyxanthate) de l'ordre de 24 000 contre une Mω (POE initial) de l'ordre de 3400.

**EXEMPLE 6 :**

Cet exemple est tout à fait semblable à l'exemple 5 sauf en ce gui concerne les quantités mises en jeu gui sont beaucoup plus importantes. Ces quantités sont les suivantes :

- POE ($M_{POE}$ = 3400 difonctionnel) = 10 g soit 5,88 . $10^{-3}$ moles de fonctions - OH
- KOH = 1,5 g soit 2,68 $10^{-2}$ moles
- $CS_2$ = 1 ml soit 1,66 $10^{-2}$ moles
- THF = 50 ml

Après oxydation et démixion de la phase visqueuse, le solvant est évaporé et le résidu repris dans l'eau. La solution ainsi obtenue est divisée en deux. La première partie est traitée comme dans les exemples 2 et 5, par dialyse et lyophilisation ce qui conduit à un polyxanthate caractérisé par une masse moyenne Mω (polyxanthate) de l'ordre de 20800 contre une Mω (POE initial) de l'ordre de 3400.

La deuxième moitié est purifiée par ultrafiltration. Le résidu de filtration et le filtrat sont lyophilisés. Les deux solides obtenus sont analysés par G.P.C.. On obtient une masse moyenne Mω (résidu) proche de 30200 pour une Mω (filtrat) voisine de 6 800. L'ultrafiltration est donc efficace.

**EXEMPLE 7** :

On répète le mode opératoire de l'exemple 5 mais avec un POE intial d'une masse moléculaire plus importante. Les quantités mises en jeu sont présentées ci-dessous :

- POE ($M_{POE}$ = 8000, difonctionnel) = 2 g soit 5 $10^{-4}$ moles de fonctions - OH
- KOH = 0,3 g soit 5,36 $10^{-3}$ moles
- $CS_2$ = 0,2 ml soit 3,32 $10^{-3}$ moles
- THF = 10 ml

Après récupération par dialyse et lyophilisation, on obtient un polyxanthate caractérisé par un chromatogramme montrant une double distribution dont :

- 1er pic : $M_{pic1}$ = 9600
- 2ème pic : $M_{pic2}$ = 22000

Le couplage a donc bien lieu.

**EXEMPLE 8** :

Le polymère est obtenu selon le mode opératoire décrit en exemple 7, en utilisant à titre de solvant le toluène en remplacement du T.H.F..

Les quantités de produits de départ, mises en oeuvre, sont les suivantes:

- POE ( MPOE = 3000 )= 30g soit 1,5 $10^{-2}$ moles de fonctions OH.
- KOH = 6g soit 1,1 $10^{-1}$ mole.
- $CS_2$ = 3,8g soit 5,0 10-2 moles.
- I2 = 7g soit 2,8 $10^{-2}$ moles.
- Toluène = 100ml pour dissoudre le POE et 140ml pour dissoudre $I_2$.

Dans cet essai, la potasse est simplement broyée et l'iode, en solution dans le toluène, est ajouté globalement au milieu réactionnel. L'oxydation qui est laissée se dérouler une vingtaine d'heures, aboutit à la décantation d'une phase visqueuse qui est traitée comme suit:

On ajoute 100ml d'eau pour solubiliser la phase visqueuse. L'ensemble est décanté et la phase aqueuse récupérée. La phase de toluène est lavée plusieurs fois avec de l'eau (environ 150ml). Toutes les fractions aqueuses sont ensuite rassemblées et la solution ainsi obtenue, analysée par GPC sans procéder à une purification quelconque. Le chromatogramme montre une double distribution.

1er pic: Mi 3000
2nd pic: Mf 4400

**EXEMPLE 9 :**

Dans cet essai, l'alcoolate est obtenu par une réaction d'oxydo-réduction et l'étape d'oxydation ultérieure est effectuée avec l'iode. Le mode opératoire mis en oeuvre est le suivant:
5g de POE ( MPOE= 2400 )(2,9 $10^{-3}$ moles de fonctions OH) sont solubilisés dans 20ml de toluène. 0,11g d'hydrure de sodium ( 4,6 $10^{-3}$ moles), préalablement lavés avec 10ml de toluène, sont introduits progressivement sous forme de poudre dans cette solution. L'ensemble est conservé à 35°C pendant une quinzaine d'heures. Après refroidissement, 0,68g de $CS_2$ (8,9 $10^{-3}$ moles) y sont ajoutés. La réaction est laissée se poursuivre pendant environ 1 heure. 0,42g d'iode (1,7 $10^{-3}$ moles) en solution dans 72ml de toluène, y sont ensuite incorporés en une seule fois. Au terme d'une vingtaine d'heures, la phase visqueuse apparue est traitée selon le mode opératoire décrit en exemple 8. L'analyse GPC en phase aqueuse du produit final révèle un couplage efficace puisque l'on note:

1er pic: Mi = 2 400
2nd pic: Mf = 10 900

**EXEMPLE 10**

Cet essai est réalisé selon les conditions de l'exemple précédent mais en mettant en oeuvre, à titre d'oxydant, l'hypochlorite de tert-butyle. Dans ce cas, pour procéder à l'oxydation, on ajoute progressivement au milieu réactionnel 0,15g d'hypochlorite de tert-butyle ( 1,4 $10^{-2}$ moles) en solution dans 15ml de toluène. la réaction d'oxydation est laissée se poursuivre une vingtaine d'heures à température ambiante. Le milieu demeure monophasique pendant l'oxydation. Le polyxanthate est extrait du toluène par lavages successifs avec de l'eau. l'analyse par GPC conduit aux résultats suivants:

- $1^{er}$ pic : Mi = 2400
- $2^{nd}$ pic : Mf = 6400

**D - PREPARATION D'UN "MACROXANTHATE".**

**EXEMPLE 11 :**

Le POE initial est dans ce cas monofonctionnel - OH et il s'agit du $CH_3O$ - POE - OH

Les quantités utilisées sont résumées ci-dessous :

- POE = ($M_{POE}$ = 2000 monofonctionnel) = 2 g soit $10^{-3}$ moles de fonctions OH
- KOH = 0,2 g soit 3,57.$10^{-3}$ moles
- $CS_2$= 0,2 ml soit 3,32 $10^{-3}$ moles
- THF = 10 ml

Le mode opératoire est identique à celui décrit dans l'exemple 5.

L'oxydation est très lente et est poursuivie jusqu'à au moins l'apparition de la phase visqueuse qui décante. Pour cet essai, l'étape d'oxydation a nécessité une dizaine d'heures.

Le polymère obtenu est caractérisé par une masse moyenne $M\omega$ (macroxanthate) proche de 3000 contre une masse $M\omega$ (POE initial) de l'ordre de 2000.

Il s'agit d'un mélange de macroxanthate et de POE non couplé.

**EXEMPLE 12 :**

L'exemple 12 est identique à l'exemple 11 si ce n'est que la quantité de potasse utilisée est plus importante :

Elle est de 0,3 g soit 5,36.$10^{-3}$ moles.

De plus, l'oxydation est poursuivie même après l'apparition de la phase visqueuse. La durée totale requise pour l'opération atteint une vingtaine d'heures.

Le polymère ainsi obtenu est mieux couplé puisqu'il est caractérisé par une masse moyenne $M\omega$ (macroxanthate) proche de 3600 contre une $M\omega$ (POE initial) d'environ 2000.

**E - POLYMERISATION**

**EXEMPLES 13 ET 14:**

Deux polymérisation en masse du méthacrylate de méthyle à 80°C en tubes scellés sont effectuées. La concentration en AIBN, utilisé comme amorceur, est de 8,3.$10^{-4}$ mol/l. Le volume total du milieu réactionnel est de 12 ml, soit une masse d'AIBN de 1, 6 mg. La concentration en polyxanthate (produit de l'exemple 6) est différente pour chaque essai.

Le mélange méthacrylate de méthyle - AIBN - polyxanthate (exemple 6) est introduit dans les tubes et débarrassé de l'oxygène dissous par une série de cycles congélation - dégazage - décongélation. Les tubes sont ensuite scellés sous vide et plongés dans un bain d'huile à 80°C où ils sont conservés 42 mn.

Le polymère formé est alors récupéré par précipitation dans le méthanol, filtration et séchage. Il est analysé par G.P.C..

Le filtrat est concentré par évaporation et le polyxanthate résiduel qui s'y trouve en est extrait par précipitation dans l'éther.

Les résultats sont rassemblés dans le tableau I ci-dessous. [polyxanthate]o y figure la concentration exprimée en moles de liens disulfure de xanthate.

TABLEAU I

| Ex | [Polyxanthate]o (mol/l) | X(%) | Mω | Mn | $X_{px}$ (%) |
|---|---|---|---|---|---|
| 13 | $8,5 \times 10^{-4}$ | 13,2 | 705 000 | 496 000 | 64,0 |
| 14 | $4,1 \times 10^{-3}$ | 9,2 | 748 000 | 496 000 | 21,0 |

**Examples 15 à 18 :**

Ces essais sont semblables dans leur déroulement à ceux décrits dans les exemples 13 et 14. Seule la concentration en AIBN qui est de $5.10^{-4}$ mol/l est différente.

Des prélèvements au cours du temps pour chaque concentration en polyxanthate (produit de l'exemple 6) sont effectués.

Les résultats sont rassemblés dans le tableau II ci-après, avec [Px]o ayant la définition explicitée en tableau I:

TABLEAU II

| Ex | $[Px]_o$ (mol/l) | t(mn) | X (%) | Mω | Mn | $R_{po}$ ($mol^{-1}s^{-1}$) | $X_{px}$ (%) |
|---|---|---|---|---|---|---|---|
| | | 20 | 3,7 | 886 000 | 464 000 | | 16,3 |
| | | 40 | 8,6 | 1032 000 | 708 000 | | 58,2 |
| 15 | $5.10^{-4}$ | 60 | 12,0 | 1179 000 | 735 000 | $3,37.10^{-4}$ | 42,4 |
| | | 80 | 16,7 | 1273 000 | 711 000 | | 39,1 |
| | | 100 | 21,7 | 1436 000 | 635 000 | | 34,8 |
| | | 120 | 31,4 | 2243 000 | 828 000 | | 34,8 |
| | | 20 | 3,4 | 1028 000 | 767 000 | | 68,5 |
| | | 40 | 7,3 | 1441 000 | 1043 000 | | 64,1 |
| 16 | $10^{-3}$ | 60 | 12,2 | 906 000 | 523 000 | $3,12.10^{-4}$ | 70,0 |
| | | 80 | 15,6 | 1045 000 | 543 000 | | 64,8 |
| | | 100 | 21,8 | 1331 000 | 752 000 | | 65,0 |
| | | 120 | 31,0 | 1852 000 | 725 000 | | 55,0 |
| | | 20 | 2,2 | 403 000 | 197 000 | | 53,7 |
| | | 40 | 7,6 | 672 000 | 439 000 | | 63,4 |
| 17 | $2,5.10^{-3}$ | 60 | 10,0 | 816 000 | 482 000 | $2,84.10^{-4}$ | 61,3 |
| | | 80 | 13,6 | 900 000 | 570 000 | | 59,1 |
| | | 100 | 18,9 | 1070 000 | 629 000 | | 69,6 |
| | | 120 | 50,3 | 1864 000 | 704 000 | | 61,0 |
| | | 20 | 0,3 | - | - | | 54,0 |
| | | 40 | 2,5 | 298 000 | 149 000 | | 65,1 |
| 18 | $5.10^{-3}$ | 60 | 5,6 | 713 000 | 465 000 | $2,5.10^{-4}$ | 66,0 |
| | | 80 | 9,2 | 755 000 | 352 000 | | 53,2 |
| | | 100 | 13,1 | 850 000 | 429 000 | | 56,7 |
| | | 120 | 16,0 | 995 000 | 456 000 | | 58,6 |

**Revendications**

**1.** Composé chimique représenté par les formules générales

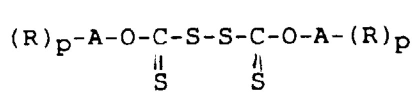

(I)

ou

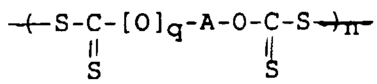 (II)

dans lesquelles
A représente:

- une séquence polymère issue d'au moins un monomère choisi parmi les monomères vinyliques et diéniques, auquel cas p vaut zéro et q est égal à 1 ou bien
- une séquence polyoxyalkylène, chaque chaînon alkylène ayant au plus 6 atomes de carbone, auquel cas p est égal à 1 et q vaut zéro, avec n variant entre 2 à 30 et de préférence entre 3 et 15 et R représentant un reste alkyle, cycloalkyle ou aryle.

**2.** Composé chimique selon la revendication 1 caractérisé en ce que A représente une séquence polyoxyalkylène.

**3.** Composé chimique selon la revendication 2 caractérisé en ce que la séquence polyoxyalkylène est choisie parmi les séquences des formules suivantes :

$$-\!\!(\!-O-CH_2-CH_2-\!)_r-$$

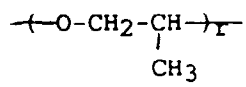

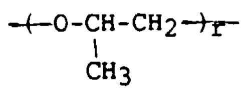

et

$$-\!\!(\!-O-(CH_2)_4-\!)_r-$$

où r vaut de 1 à 1000 et de préférence de 1 à 200.

**4.** Composé chimique selon la revendication 1, 2 ou 3 caractérisé en ce que A représente un polyoxyde d'éthylène dont la masse moléculaire varie entre 1000 et 10 000 et est en particulier proche de 3000.

**5.** Procédé de préparation d'un composé selon l'une quelconque des revendications 1 à 4 caractérisé en ce que l'on fait réagir un alcoolate de formules générales:

$$(R)_p\text{-}A\text{-}O^{\ominus}(X) \qquad \text{(Ibis)}$$

ou

$$(X)^{\ominus}(O)_q\text{-}A\text{-}O^{\ominus}(X) \qquad \text{(IIbis)}$$

dans lesquelles A, R, p et q ont les significations ou valeurs explicitées en revendication 1 et X représente un sel métallique et de préférence un atome de potassium ou de sodium, sur le disulfure de carbone, en présence d'un

agent oxydant ou en ajoutant cet agent à la fin de la réaction.

**6.** Procédé selon la revendication 5 caractérisé en ce que l'oxydant est l'iode ou l'hypochlorite de tert-butyle.

**7.** Procédé selon la revendication 5 ou 6 caractérisé en ce que l'alcoolate peut être obtenu par réaction d'un polymère de formule générale :

$$(R)_p\text{-A- OH} \qquad \text{(Iter)}$$

ou

$$\text{H-(O)}_q\text{-A-OH} \qquad \text{(IIter)}$$

avec A, R, p et q tels que définis en revendication 1, en milieu hétérogène avec un fort excès d'une base forte.

**8.** Procédé selon la revendication 7 caractérisé en ce que la base utilisée est la potasse ou la soude.

**9.** Procédé selon la revendication 7 ou 8 caractérisé en ce que le macro- ou polyxanthate obtenu peut être purifié par dialyse ou par ultrafiltration.

**10.** Produit susceptible d'être obtenu par mise en oeuvre d'un procédé tel que défini à l'une quelconque des revendications 5 à 9.

**11.** Utilisation d'un composé selon l'une quelconque des revendications 1 à 4 comme agent ayant la fonction d'agent de transfert de chaîne et d'agent de terminaison dans la polymérisation radicalaire de monomères vinyliques en milieu monophasique.

**12.** Utilisation selon la revendication 11 caractérisé en ce que le monomère vinylique est un monomère acrylique, méthacrylique ou styrène.

**13.** Polymères vinyliques susceptibles d'être obtenus par polymérisation radicalaire d'au moins un monomère vinylique en milieu monophasique et en présence d'au moins un composé selon l'une des revendications 1 à 4.

**Patentansprüche**

**1.** Chemische Verbindung, die durch die allgemeinen Formeln

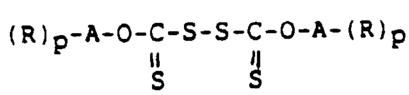

(I)

oder

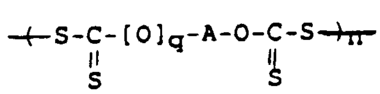

(II)

wiedergegeben wird, in denen
A:

- für eine polymere Sequenz, die von mindestens einem Monomer stammt, das ausgewählt wird aus den Vinylmonomeren und den Dienmonomeren, in welchem Falle p Null und q gleich 1 ist, oder auch

- für eine Polyoxyalkylensequenz steht, bei der jedes Alkylenkettenglied höchstens 6 Kohlenstoffatome aufweist, in welchem Falle p gleich 1 und q gleich Null ist, wobei n zwischen 2 und 30 und vorzugsweise zwischen 3 und 15 variiert und R für einen Alkyl-, Cycloalkyl- oder Arylrest steht.

**2.** Chemische Verbindung nach Anspruch 1, dadurch gekennzeichnet, daß A für eine Polyoxyalkylensequenz steht.

**3.** Chemische Verbindung nach Anspruch 2, dadurch gekennzeichnet, daß die Polyoxyalkylensequenz ausgewählt wird unter den Sequenzen der folgenden Formeln:

$$-(-O-CH_2-CH_2-)_r-$$

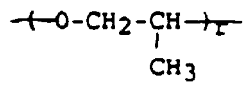

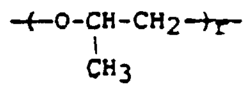

und

$$-(-O-(CH_2)_4-)_r-$$

in denen r 1 bis 1000 und vorzugsweise 1 bis 200 beträgt.

**4.** Chemische Verbindung nach Anspruch 1,2 oder 3, dadurch gekennzeichnet, daß A für ein Polyethylenoxid steht, dessen Molekülmasse zwischen 1000 und 10000 beträgt und insbesondere nahe bei 3000 liegt.

**5.** Verfahren zur Herstellung einer Verbindung nach einem der Ansprüche 1 bis 4, dadurch gekennzeichnet, daß man ein Alkoholat der allgemeinen Formeln

$$(R)_p\text{-A-O}^{\ominus}(X) \qquad (I.2)$$

oder

$$(X)^{\ominus}(O)_q\text{-A-O}^{\ominus}(X) \qquad (II.2),$$

in denen A, R, p und q die in Anspruch 1 angegebenen Bedeutungen oder Werte haben und X für ein Metallsalz und vorzugsweise ein Kaliumatom oder ein Natriumatom steht, mit Kohlenstoffdisulfid reagieren läßt in Gegenwart eines Oxidationsmittels, oder indem man dieses Mittel am Ende der Reaktion zusetzt.

**6.** Verfahren nach Anspruch 5, dadurch gekennzeichnet, daß das Oxidationsmittel Iod oder tert.-Butylhypochlorit ist.

**7.** Verfahren nach Anspruch 5 oder 6, dadurch gekennzeichnet, daß das Alkoholat durch Umsetzung eines Polymers der allgemeinen Formel:

$$(R)_p\text{-A- OH} \qquad (I.3)$$

oder

$$H\text{-}(O)_q\text{-A-OH} \qquad (II.3),$$

wobei A, R, p und q wie in Anspruch 1 definiert sind, in einem heterogenen Medium mit einem hohen Überschuß einer starken Base erhalten werden kann.

**8.** Verfahren nach Anspruch 7,
dadurch gekennzeichnet,
daß die verwendete Base Kaliumhydroxid oder Natriumhydroxid ist.

**9.** Verfahren nach Anspruch 7 oder 8,
dadurch gekennzeichnet,
daß das erhaltene Makro- oder Polyxanthat durch Dialyse oder durch Ultrafiltration gereinigt werden kann.

**10.** Erzeugnis, das durch Ausführen eines Verfahrens, wie es in einem der Ansprüche 5 bis 9 definiert ist, erhalten werden kann.

**11.** Verwendung einer Verbindung nach einem der Ansprüche 1 bis 4 als Mittel, das die Funktion eines Kettenübertragungsmittels und eines Kettenabbruchmittels in der radikalischen Polymerisation von Vinylmonomeren in einphasigem Medium aufweist.

**12.** Verwendung nach Anspruch 11,
dadurch gekennzeichnet,
daß das Vinylmonomer ein acrylisches, methacrylisches oder styrolisches Monomer ist.

**13.** Vinylpolymere, die durch radikalische Polymerisation von mindestens einem Vinylmonomer in einphasigem Medium und in Gegenwart von mindestens einer Verbindung nach einem der Ansprüche 1 bis 4 erhalten werden können.

## Claims

**1.** A chemical compound represented by the following general formulae:

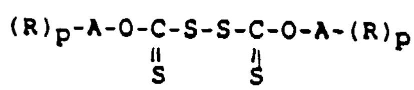

(I)

or

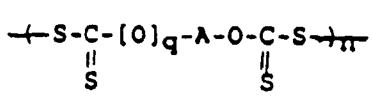

(II)

in which:
A represents:

- a polymer sequence resulting from at least one monomer selected from vinyl and diene monomers, in which case p is zero and q is equal to 1 or
- a polyoxyalkylene sequence, each alkylene chain link having at most 6 carbon atoms, in which case p is equal

to 1 and q is zero, with n varying between 2 and 30 and preferably between 3 and 15 and R representing an alkyl, cycloalkyl or aryl residue.

2. A chemical compound according to claim 1 characterised in that A represents a polyoxyalkylene sequence.

3. A chemical compound according to claim 2 characterised in that the polyoxyalkylene sequence is selected from sequences of the following formulae:

$$-(-O-CH_2-CH_2-)_r-$$

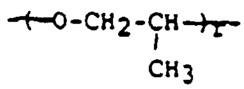

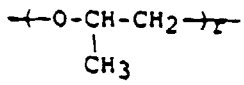

and

$$-(-O-(CH_2)_4-)_r-$$

wherein r is from 1 to 1000 and preferably from 1 to 200.

4. A chemical compound according to claim 1, claim 2 or claim 3 characterised in that A represents a polyethylene oxide whose molecular mass varies between 1000 and 10,000 and is in particular close to 3000.

5. A process for the preparation of a compound according to any one of claims 1 to 4 characterised by reacting an alcoholate of the following general formulae:

$$(R)_p\text{-A-}O^{\ominus}(X) \qquad \text{(Ibis)}$$

or

$$(X)^{\ominus}(O)_q\text{-A-}O^{\ominus}(X) \qquad \text{(IIbis)}$$

in which A, R, p and q bear the meanings or values recited in claim 1 and X represents a metal salt and preferably a potassium or sodium atom, on carbon disulphide, in the presence of an oxidising agent or with the addition of said agent at the end of the reaction.

6. A process according to claim 5 characterised in that the oxidising agent is iodine or tert-butyl hypochlorite.

7. A process according to claim 5 or claim 6 characterised in that the alcoholate can be obtained by the reaction of a polymer of the general formula:

$$(R)_p\text{-A-OH} \qquad \text{(Iter)}$$

or

$$H\text{-}(O)_q\text{-A-OH} \qquad \text{(IIter)}$$

with A, R, p and q as defined in claim 1, in a heterogeneous medium, with a large excess of a strong base.

**8.** A process according to claim 7 characterised in that the base used is potassium hydroxide or sodium hydroxide.

**9.** A process according to claim 7 or claim 8 characterised in that the macro- or polyxanthate can be purified by dialysis or by ultrafiltration.

**10.** A product which can be obtained by carrying out a process as defined in any one of claims 5 to 9.

**11.** Use of a compound according to any one of claims 1 to 4 as an agent having the function of a chain transfer agent and a termination agent in the radical polymerisation of vinyl monomers in a single-phase medium.

**12.** Use according to claim 11 characterised in that the vinyl monomer is an acrylic, methacrylic or styrene monomer.

**13.** Vinyl polymers which can be obtained by radical polymerisation of at least one vinyl monomer in a single-phase medium and in the presence of at least one compound according to one of claims 1 to 4.